(19) Europäisches Patentamt
European Patent Office
Office européen des brevets

(11) Veröffentlichungsnummer : **0 073 000**
**B1**

(12) # EUROPÄISCHE PATENTSCHRIFT

(45) Veröffentlichungstag der Patentschrift : **26.06.85**

(51) Int. Cl.⁴ : **C 07 C161/00**, C 07 D235/26

(21) Anmeldenummer : **82107469.7**

(22) Anmeldetag : **17.08.82**

(54) **Verfahren zur Herstellung von Arylhydrazin-N-sulfonsäuren.**

(30) Priorität : **21.08.81 DE 3133100**

(43) Veröffentlichungstag der Anmeldung : **02.03.83 Patentblatt 83/09**

(45) Bekanntmachung des Hinweises auf die Patenterteilung : **26.06.85 Patentblatt 85/26**

(84) Benannte Vertragsstaaten : **BE CH DE FR GB IT LI**

(56) Entgegenhaltungen :
**CHEMISCHES ZENTRALBLATT, Band 1929II, Nr. 9, 28. August 1929, Seiten 1656-1657 Mc CLURE et al. "Elektrochemische Darstellung des Phenylhydrazins"**

(73) Patentinhaber : **HOECHST AKTIENGESELLSCHAFT**
**Postfach 80 03 20**
**D-6230 Frankfurt am Main 80 (DE)**

(72) Erfinder : **Berthold, Rüdiger, Dr.**
**Geierfeld 55**
**D-6232 Bad Soden am Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 073 000 B1

**Beschreibung**

Arylhydrazin-N-sulfonsäuren lassen sich leicht zu den entsprechenden Arylhydrazinen verseifen und sind somit wichtige Zwischenprodukte für diese vielseitig verwendbaren Verbindungen.

Es ist bekannt, daß man Arylhydrazin-N-sulfonsäuren durch Reduktion der entsprechenden Aryldiazosulfonate herstellen kann. Nach Trans. Am. electrochem. Soc. *56,* 452 ; C 1929 II 1657, kann diese Reduktion mit Zinkstaub in Gegenwart von Säure, mit Zinn und Salzsäure, aber auch elektrochemisch an einer Quecksilberkathode erfolgen. In der Praxis wird hierfür Natriumhydrogensulfit eingesetzt. Hierbei entsteht jedoch aus dem umgesetzten Sulfit das Hydrogensulfat bzw. nach der Neutralisation das Sulfat und aus dem im Überschuß eingesetzen Sulfit Schwefeldioxid. Da Sulfat bei der Abwasseraufbereitung den Beton der Kläranlagen angreift, ist eine entsprechend aufwendige Aufbereitung der Abwässer erforderlich.

Es wurde auch schon beschrieben, Schwefeldioxid als Reduktionsmittel einzusetzen (Die Chemie 56 (1943) 233). Trotz guter Ausbeuten hat sich dieses Verfahren nicht in die Praxis eingeführt, da es — insbesondere wegen seiner schlechten Raumausbeute — recht unwirtschaftlich arbeitet. So müssen für eine Tonne Phenylhydrazin bei einem Reaktionsvolumen von 113 $m^3$, was etwa 150 $m^3$ Kessel-volumen entspricht, 86 $m^3$ Wasser abdestilliert werden.

Ein weiterer Nachteil des bekannten Verfahrens liegt darin, daß substituierte Arylhydrazine nach diesem Verfahren entweder überhaupt nicht oder nur mit schlechten Ausbeuten zugänglich sind (Houben-Weyl 10/2, 190 ; J. Am. Chem. Soc. 78 (1956) 5856).

Es wurde nun gefunden, daß die vorstehend genannten Nachteile vermieden werden, wenn man die Aryldiazosulfonate katalytisch hydriert. Gegen diese Art der Reduktion bestand offenbar ein Vorurteil, da bei der Vielzahl der hierfür vorgeschlagenen Reduktionsmittel (Houben-Weyl, a.a.O. 180 bis 223) die katalytische Hydrierung nicht in Betracht gezogen wurde, vermutlich deshalb, weil Reduktionsmittel, die eine Nitrogruppe in die Aminogruppe überführen können, aromatische Hydrazoverbindungen zu den Aminen spalten (Houben Weyl 11/1, 522). So disproportioniert Hydrazobenzol in Gegenwart von Palladiumschwarz bereits ohne Wasserstoff in Anilin und Azobenzol. Die Hydrierung in Gegenwart von Nickel liefert Anilin in praktisch quantitativer Ausbeute. Unter denselben Bedingungen zerfällt Phenylhydrazin leicht in Anilin und Amoniak (Houben-Weyl, a.a.O. 538).

Die Erfindung betrifft somit ein Verfahren zur Herstellung von Verbindungen der allgemeinen Formel I

$$R_nAr—NH—NH—SO_3M \qquad (I)$$

in welcher Ar für einen aromatischen Isocyclus oder Heterocyclus steht, R gleich oder verschieden ist und für Alkyl, Aryl, Amino, Hydroxy, Alkoxy, Aryloxy, Halogen, Nitro, Acylamino, Carbamoyl, Alkylcarbamoyl, Arylcarbamoyl, Dialkylamino, Arylamino, Sulfo oder Sulfamoyl, M für Wasserstoff, Alkalimetall oder ein Äquivalent Erdalkalimetall und n für 0 oder eine ganze Zahl von 1 bis 5 steht, durch Reduktion von Verbindungen der allgemeinen Formel II

$$R_nAr—N = N—SO_3M \qquad (II)$$

in welcher Ar, R, M und n die vorstehend genannten Bedeutungen haben, in einem Lösungsmittel, das dadurch gekennzeichnet ist, daß die Reduktion mit Wasserstoff in Gegenwart von Übergangsmetallkatalysatoren durchgeführt wird.

Ein Vorzug des erfindungsgemäßen Verfahrens ist, daß die katalytische Hydrierung sehr selektiv ausgeführt werden kann, so daß beispielsweise Nitrogruppen im Edukt der Formel II wahlweise erhalten bleiben oder in Aminogruppen überführt werden können. Dementsprechend ist in einer bevorzugten Ausführungsform der Erfindung mindestens ein Rest R sowohl im Edukt der Formel II als auch im Produkt der Formel I Nitro bzw. mindestens ein Rest R im Edukt Nitro und mindestens ein Rest R im Produkt Amino.

Allgemein sind bevorzugte Edukte der Formel II und Produkte der Formel I solche Verbindungen, in denen Ar für Phenyl steht, R für niederes Alkyl, niederes Alkoxy, Chlor, Brom, Nitro, Phenyl, Amino, Acylamino, insbesondere Alkanoylamino und Benzoylamino, Carbamoyl, niederes Dialkylamino, Phenylamino, Sulfo- oder Sulfamoyl, M für Wasserstoff oder Alkalimetall und n für 0 bis 3 stehen.

Im folgenden werden verfahrensmäßig bevorzugte Ausführungen der Erfindung näher erläutert :

Als Übergangsmetallkatalysatoren eignen sich die üblichen Hydrierkatalysatoren auf Basis der Metalle Nickel, Kobalt, Platin, Palladium, Ruthenium, Rhodium oder Iridium. Bevorzugt sind Platin und Palladium. Diese Metalle werden im allgemeinen auf einem Träger eingesetzt, beispielsweise auf Kohle, Siliziumdioxid, Aluminiumoxid, Aluminiumsilikaten, Spinellen und Zeolithen. Hierbei enthält der Katalysator zweckmäßig 0,05 bis 10, vorzugsweise 0,2 bis 5, insbesondere 0,5 bis 2,5 Gew.-% Metall. Es können aber auch Skelettkatalysatoren verwendet werden, beispielsweise Raney-Nickel.

Da sowohl die Edukte als auch die Produkte mehr oder weniger gut wasserlöslich sind, ist Wasser das bevorzugte Reaktionsmedium. In besonderen Fällen kommen auch wassermischbare Lösungsmittel,

beispielsweise niedere Alkohole wie Methanol und Ethanol, sowie Dimethylformamid und N-Methylpyrrolidon bzw. deren Gemische mit Wasser in Betracht. Es ist auch möglich, die Aryldiazosulfonate in Suspension in hydrophoben Lösungsmitteln zu hydrieren.

Die Hydrierung kann sowohl bei Normaldruck als auch bei erhöhtem Druck durchgeführt werde, wobei die Reaktionsgeschwindigkeit mit dem Druck zunimmt. In der Praxis kommen Drücke bis 300 bar in Betracht, wobei Drücke bis 100 bar und insbesondere ein Druckbereich von 20 bis 60 bar bevorzugt sind.

Die Temperatur kann in weiten Bereichen gewählt werden. Sie hängt von der Beständigkeit des Diazosulfonats und von der gewünschten Reaktionsgeschwindigkeit ab. Zweckmäßig wird die Reaktionstemperatur so gewählt, daß Edukt und Produkt in Lösung bleiben. Im allgemeinen wird man deshalb bei Temperaturen von etwa 0 bis 200 °C, vorzugsweise 20 bis 120 °C, insbesondere bei etwa 30 bis 80 °C arbeiten.

Zur Erzielung einer guten Raum-Zeit-Ausbeute wird die Konzentration des Eduktes möglichst hoch gewählt und im Einklang mit der Beständigkeit des Edukts und der Löslichkeit von Edukt und Produkt die übrigen Parameter eingestellt.

Des erfindungsgemäße Verfahren läßt sich auch kontinuierlich durchführen.

Es is auch möglich, das Edukt in situ herzustellen, wobei die Arylhydrazin-N-sulfonsäuren aus dem entsprechenden aromatischen Amin durch Diazotieren und Umsetzen mit Sulfit gewonnen werden. Dieses Verfahren ist besonders vorteilhaft, wenn die Arylhydrazin-N-sulfonsäure gut wasserlöslich ist, Hierbei wird bevorzugt die wäßrige Lösung des Diazoniumsalzes mit einer Lösung oder Suspension eines Alkalimetall- oder Erdalkalimetallsulfits in der Weise zur Reaktion gebracht, daß man einen Überschuß eines der beiden Reaktionspartner während der Reaktion weitgehend ausschließt, beispielsweise dadurch, daß man die Diazoniumsalzlösung so rasch wie technisch möglich zur sehr schnell gerührten Lösung oder Suspension des Sulfits gibt oder daß man stöchiometrische Mengen der Lösungen bzw. Suspensionen der Reaktionspartner kontinuierlich in einen Durchlaufmischer dosiert und anschließend in ein rührbares Verweilgefäß leitet. Ein solches Verfahren ist in der deutschen Patentanmeldung P 31 25 104.8 beschrieben.

Die Produkte können in bekannter Weise zum entsprechenden Arylhydrazin bzw. zu dessen Salzen verseift werden. Bei gut löslichen bzw. schwer isolierbaren Produkten ist es vorteilhaft, diese Hydrolyse unmittelbar an das erfindungsgemäße Verfahren anzuschließen, ohne die Arylhydrazin-N-sulfonsäure zu isolieren.

In den folgenden Beispielen beziehen sich Prozentangaben auf das Gewicht, sofern nichts anderes angegeben ist.

### Beispiel 1

Natriumsalz der Phenylhydrazin-N-sulfonsäure

277 g 75 %iges Natriumsalz der Phenyldiazosulfonsäure (entsprechend 208 g 100 %iges Produkt = 1,0 Mol) wurden bei 55 °C in 700 ml Wasser gelöst. Die rotbraun gefärbte Lösung klärteman mit 5 g Aktivkohle und gab 5 g Natriumcarbonat (wasserfrei) zu.

Unter Zusatz von 5 g Platin auf Kohlenstoff (5 % Platin) hydrierte man bei 80 °C und 20 bis 40 bar Wasserstoffdruck, bis kein Druckabfall mehr feststellbar war. Dies war nach etwa 10 Minuten der Fall. Die hydrierte, schwach gelbliche Lösung wurde bei 80 °C vom Katalysator abgesaugt und mit 100 g Kochsalz versetzt. Das ausgesalzene Produkt wurde bei + 5 °C abgesaugt und getrocknet. Man erhielt 217 g Natriumsalz der Phenylhydrazin-N-sulfonsäure mit einem Reingehalt von 89 %, entsprechend 92 % der Theorie.

### Beispiel 2

Phenylhydrazin-Hydrochlorid

In diesem Beispiel wird gezeigt, wie die entstehende Phenylhydrazin-N-sulfonsäure zum Salz des Phenylhydrazins verseift wird.

550 g Natriumsalz der Phenyldiazosulfonsäure mit einem Reingehalt von 82,8 % (entsprechend 455 g 100 %iges Produkt = 2,19 Mol) wurden bei 40 °C in 2,8 l Wasser gelöst und die rotbraun gefärbte Lösung mit 10 g Aktivkohle geklärt. Unter Zusatz von 5 g Platinkatalysator (5 % Platin auf Kohle) hydrierte man die Lösung, die einen pH-Wert von 10 zeigt, bei 40 °C und 40 bar Wasserstoffdruck, bis kein Druckabfall mehr feststellbar war. Dies war nach etwa 10 Minuten der Fall. Nach dem Absaugen vom Katalysator versetzte man das Filtrat (2,85 l) mit 400 g 30 %iger Salzsäure und destillierte innerhalb von 1 Stunde 1,5 l Wasser ab. Nach Abkühlen auf 5 °C wurde der Niederschlag abgesaugt. Man erhielt 348 g Phenylhydrazin-Hydrochlorid mit einem Reingehalt von 81 %, entsprechend 89 % der Theorie.

### Beispiel 3

Natriumsalz der p-Methoxyphenylhydrazin-N-sulfonsäure

120 g Natriumsalz der p-Methoxyphenyldiazosulfonsäure vom Reingehalt 99,2 % und 5 g Natriumcarbonat (wasserfrei) wurden bei 80 °C in 300 ml Wasser gelöst und mit 5 g Aktivkohle geklärt. Die geklärte Lösung wurde bei 30 bis 40 °C in Gegenwart von 5 g Raney-Nickel unter 40 bar Wasserstoffdruck hydriert. Es wurde die theoretische Menge Wasserstoff verbraucht. Man erwärmte, bis das entstandene Hydrazosulfonat in Lösung gegangen war und saugte vom Katalysator ab. Aus dem Filtrat erhielt man nach Abkühlen auf 5 °C beim Absaugen 40 g weißes trockenes Hydrazosulfonat. Die Mutterlauge wurde im Wasserstrahlvakuum zur Trockene eingeengt. Dadurch konnte weitere 68,6 g beige-bräunliches Hydrazosulfonat gewonnen werden. Die Gesamtausbeute betrug 108,6 g Natriumsalz der p-Methoxyphenylhydrazin-N-sulfonsäure mit einem Reingehalt von 99,5 %, entsprechend 90 % der Theorie.

Verwendet man anstelle des Raney-Nickels einen Nickel-Trägerkatalysator, so ist es erforderlich, den Ansatz auf etwa 80 °C zu erwärmen, damit die Hydrierung anspringt.

## Beispiel 4

Natriumsalz der p-Nitrophenylhydrazin-N-sulfonsäure

In diesem Beispiel wird gezeigt, wie die Azogruppe selektiv hydriert wird, ohne daß die Nitrogruppe angegriffen wird.

132 g Natriumsalz der p-Nitrophenyldiazosulfonsäure (Reingehalt 97 %) und 300 ml Wasser wurden in einem 1 l Autoklav bei Raumtemperatur in Gegenwart von 5 g sulfitiertem Platinkatalysator (5 % Platin auf Kohle, gemäß Beispiel 1 der DE-PS 2 105 780) hydriert. Dabei stieg die Temperatur auf etwa 40 °C an. Es wurden 11,1 l Wasserstoff (auf 0 °C umgerechnet, 0,49 Mol) aufgenommen. Nach dem Hydrieren wurde heiß vom Katalysator abgesaugt und das Filtrat bis zum Erkalten gerührt. Man erhielt 79 g Natriumsalz der p-Nitrophenylhydrazin-N-sulfonsäure mit einem Reingehalt von 96,5 %, entsprechend 60 % der Theorie.

## Beispiel 5

Natrium-phenylhydrazin-2,4,β-N-trisulfonat

In diesem Beispiel wird gezeigt, wie das Aryldiazosulfonat in situ hergestellt und umgesetzt werden kann.

127 g 2,4-Anilindisulfonsäure (0,5 Mol) wurden in 500 ml Wasser heiß gelöst und mit 5 g Aktivkohle geklärt. Zum Filtrat gab man 10 g 31 %ige Salzsäure und kühlte auf 0 bis 5 °C ab. Der erhaltene weiße Brei wurde bei dieser Temperatur mit 88 g 40 %iger Natriumnitritlösung diazotiert. Das Reaktionsgemisch wurde in eine gut gerührte Mischung auf 69 g Natriumsulfit, 15 g 33 %iger Natronlauge und 200 ml Wasser so schnell wie möglich eingestürzt. Dabei fiel der pH-Wert von 14 auf 9,2 ab. Nach zweistündigem Nachrühren wurde nochmals mit Aktivkohle geklärt und das Filtrat in Gegenwart von 3 g Platin auf Kohle (5 % Platin) bei 25 bis 30 °C mit Wasserstoff bei ca. 40 bar hydriert. Es wurde die berechnete Menge Wasserstoff aufgenommen. Nach dem Absaugen vom Katalysator wurde das Filtrat im Vakuum eingeengt. Man erhielt 184 g Natrium-2,4,β-N-phenylhydrazin-trisulfonat (89 % der Theorie).

## Beispiele 6 bis 23

Allgemeine Verfahrensvorschrift

1 Mol Natrium-Aryldiazosulfonat (das von Zersetzungsprodukten der Diazoniumsalzlösung frei sein soll, weil diese die Aktivität des Katalysators beeinträchtigen können) wurde in einem Hydrierer mit Hubrührer in 100 ml Wasser suspendiert. Hierzu gab man 5 g Platinkatalysator (5 % Platin auf Kohle) und drückte 60 bar Wasserstoff auf. Bei etwa 20 bis 60 °C hydrierte man nun, bis kein Wasserstoff mehr aufgenommen wurde. Nach dem Entspannen des Restdruckes wurde des Gasraum des Autoklaven mit Stickstoff gespült und unter Stickstoff und Rühren auf 80 bis 90 °C erhitzt. Dabei ging im allgemeinen das entstandene Arylhydrazin-N-sulfonsäuresalz in Lösung. Die Lösung wurde aus dem Autoklaven gedrückt und vom Katalysator abgesaugt. Zum warmen Filtrat gab man gegebenenfalls die erforderliche Menge Kochsalz und rührte bis zum Erkalten. Das ausgefallene Produkt wurde bei + 5 °C abgesaugt und getrocknet.

Die Wassermenge mußte in Abhängigkeit von der Löslichkeit des Produktes manchmal etwas verändert werden. Beim Phenylamino-phenylhydrazosulfonat wurde der Katalysator zweckmäßig mehrmals mit der Mutterlauge ausgekocht.

Nach dieser Vorschrift wurden erhalten :

(Siehe Tabelle Seite 5 ff.)

| Beispiel | Produkt | Molgewicht | Ausbeute % d.Th. |
|---|---|---|---|
| 6 | 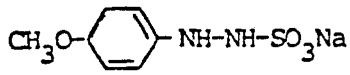 | 240 | 91 |
| 7 | 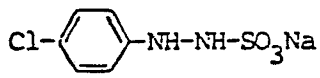 | 244,5 | 83 |
| 8 | 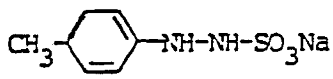 | 224 | 90 |
| 9 | 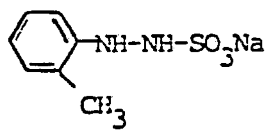 | 224 | 88 |
| 10 | 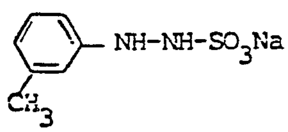 | 224 | 83 |
| 11 | 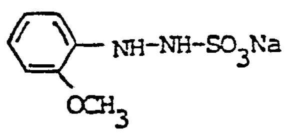 | 240 | 85 |
| 12 | 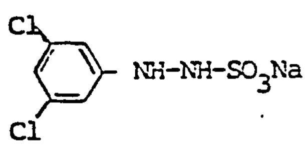 | 279 | 79 |
| 13 | 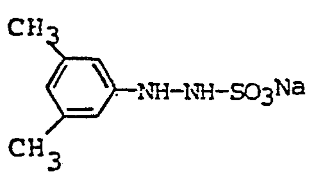 | 238 | 90 |
| 14 | 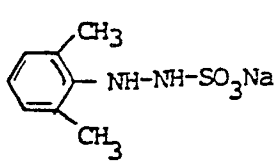 | 238 | 71 |
| 15 | 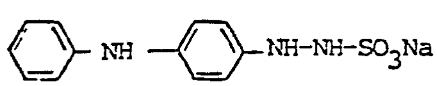 | 292 | 75 |

| Beispiel | Produkt | Molgewicht | Ausbeute % d.Th. |
|---|---|---|---|
| 16 | 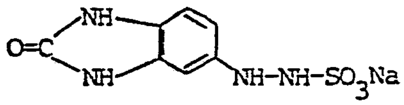 | 266 | 85 |
| 17 | 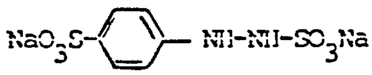 | 312 | 90 |
| 18 | 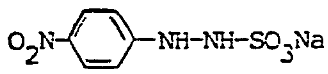 | 255 | 60 |
| 19 | 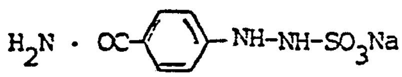 | 253 | 80 |
| 20 | 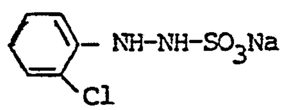 | 244,5 | 88 |
| 21 | 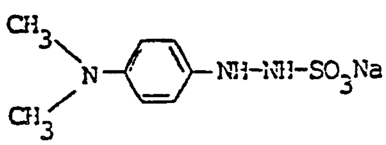 | 253 | 70 |
| 22 | 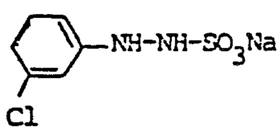 | 244,5 | 76,1 |
| 23 | 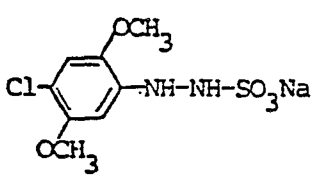 | 304,5 | 82 |

**Patentansprüche**

1. Verfahren zur Herstellung von Verbindungen der allgemeinen Formel I

$$R_nAr—NH—NH—SO_3M \qquad (I)$$

in welcher Ar für einen aromatischen Isocyclus oder Heterocyclus steht, R gleich oder verschieden ist und für Alkyl, Aryl, Amino, Hydroxy, Alkoxy, Aryloxy, Halogen, Nitro, Acylamino, Carbamoyl, Alkylcarbamoyl, Arylcarbamoyl, Dialkylamino, Arylamino, Sulfo oder Sulfamoyl, M für Wasserstoff, Alkalimetall oder ein

Äquivalent Erdalkalimetall und n für 0 oder eine ganze Zahl von 1 bis 5 stehen, durch Reduktion von Verbindungen der allgemeinen Formel II

$$R_nAr—N = N—SO_3M \quad (II)$$

in welcher Ar, R, M und n die vorstehend genannten Bedeutungen haben, in einem Lösungsmittel, dadurch gekennzeichnet, daß die Reduktion mit Wasserstoff in Gegenwart von Übergangsmetallen der 8. Nebengruppe des Periodischen Systems der Elemente als Katalysatoren durchgeführt wird.

2. Verfahren nach Anspruch 1, dadurch gekennzeichnet, daß Ar für Phenyl steht, R für niederes Alkyl, niederes Alkoxy, Chlor, Brom, Nitro, Phenyl, Amino, Acylamino, Carbamoyl, niederes Dialkylamino, Phenylamino, Sulfo oder Sulfamoyl, M für Wasserstoff oder Alkalimetall und n für 0 bis 3 stehen.

3. Verfahren nach Anspruch 1 und 2, dadurch gekennzeichnet, daß mindestens ein Rest R sowohl im Edukt der Formel II als auch im Produkt der Formel I für Nitro steht.

4. Verfahren nach Anspruch 1 und 2, dadurch gekennzeichnet, daß mindestens ein Rest R im Edukt der Formel II für Nitro und mindestens ein Rest R im Produkt der Formel I für Amino steht.

5. Verfahren nach Anspruch 1 bis 4, dadurch gekennzeichnet, daß die Hydrierung in Gegenwart von Metallen aus der 8. Nebengruppe des Periodensystems auf einem Träger durchgeführt wird.

6. Verfahren nach Anspruch 1 bis 5, dadurch gekennzeichnet, daß die Hydrierung in Wasser oder einem wassermischbaren Lösungsmittel durchgeführt wird.

7. Verfahren nach Anspruch 1 bis 6, dadurch gekennzeichnet, daß die Hydrierung bei Drücken bis 300 bar und Temperaturen von 0 bis 200 °C durchgeführt wird.

8. Verfahren nach Anspruch 1 bis 7, dadurch gekennzeichnet, daß die Hydrierung bei Drücken bis 100 bar und Temperaturen von 20 bis 120 °C durchgeführt wird.

9. Verfahren nach Anspruch 1 bis 8, dadurch gekennzeichnet, daß die Hydrierung bei Drücken von 20 bis 60 bar und Temperaturen von 30 bis 80 °C durchgeführt wird.

10. Verfahren nach Anspruch 1 bis 9, dadurch gekennzeichnet, daß die Hydrierung kontinuierlich durchgeführt wird.

**Claims**

1. A process for the preparation of compounds of the general formula I

$$R_nAr—NH—NH—SO_3M \quad (I)$$

in which Ar represents an aromatic isocycle or heterocycle, R represents identical or different alkyl, aryl, amino, hydroxyl, alkoxy, aryloxy, halogen, nitro, acylamino, carbamoyl, alkylcarbamoyl, arylcarbamoyl, dialkylamino, arylamino, sulfo or sulfamoyl, M represents hydrogen, alkali metal or one equivalent of alkaline earth metal and n represents 0 or a whole number from 1 to 5, by reduction of compounds of the general formula II

$$R_nAr—N = N—SO_3M \quad (II)$$

in which Ar, R, M and n have the abovementioned meanings, in a solvent, which comprises carrying out the reduction with hydrogen in the presence of transition metals of the 8th sub-group of the periodic system as catalysts.

2. The process as claimed in claim 1, wherein Ar represents phenyl, R represents lower alkyl, lower alkoxy, chlorine, bromine, nitro, phenyl, amino, acylamino, carbamoyl, lower dialkylamino, phenylamino, sulfo or sulfamoyl, M represents hydrogen or alkali metal and n represents 0 to 3.

3. The process as claimed in claim 1 and 2, wherein at least one radical R both in the precursor of the formula II and also in the product of the formula I represents nitro.

4. The process as claimed in claim 1 and 2, wherein at least one radical R in the precursor of the formula II represents nitro and at least one radical R in the product of the formula I represents amino.

5. The process as claimed in claims 1 to 4, wherein the hydrogenation is carried out in the presence of metals from the 8th sub-group of the periodic system on a support.

6. The process as claimed in any of claims 1 to 5, wherein the hydrogenation is carried out in water or a watermiscible solvent.

7. The process as claimed in any of claims 1 to 6, wherein the hydrogenation is carried out under pressures up to 300 bar and at temperatures from 0 to 200 °C.

8. The process as claimed in any of claims 1 to 7, wherein the hydrogenation is carried out under pressures up to 100 bar and at temperatures of 20 to 120 °C.

9. The process as claimed in any of claims 1 to 8, wherein the hydrogenation is carried out under pressures of 20 to 60 bar and at temperatures of 30 to 80 °C.

10. The process as claimed in any of claims 1 to 9, wherein the hydrogenation is carried out continously.

**Revendications**

1. Procédé de préparation de composés répondant à la formule générale I

$$R_nAr—NH—NH—SO_3M \qquad (I)$$

dans laquelle Ar représente un radical aromatique isocyclique ou hétérocyclique, les R sont identiques ou différents et représentent chacun un alkyle, un aryle, un amino, un hydroxy, un alcoxy, un aryloxy, un halogène, un nitro, un acylamino, un carbamoyle, un alkylcarbamoyle, un arylcarbamoyle, un dialkylamino, un arylamino, un sulfo ou un sulfamoyle, M représente l'hydrogène, un métal alcalin ou un équivalent d'un métal alcalino-terreux, et n désigne un nombre entier de 0 à 5, par réduction de composés répondant à la formule générale II

$$R_nAr—N = N—SO_3M \qquad (II)$$

dans laquelle Ar, R, M et n ont les significations qui viennent d'être données, dans un solvant, procédé caractérisé en ce que la réduction est effectuée au moyen d'hydrogène en présence de métaux de transition du huitième sous-groupe de la classification périodique des éléments, jouant le rôle de catalyseurs.

2. Procédé selon la revendication 1 caractérisé en ce que Ar représente un phényle, R représente un alkyle inférieur, un alcoxy inférieur, le chlore, le brome, un nitro, un phényle, un amino, un acylamino, un carbamoyle, un dialkylamino inférieur, un phénylamino, un sulfo ou un sulfamoyle, M représente l'hydrogène ou un métal alcalin, et n est un nombre de 0 à 3.

3. Procédé selon l'une des revendications 1 et 2, caractérisé en ce qu'au moins un radical R, aussi bien dans le corps de départ de formule II que dans le produit de formule I, est un radical nitro.

4. Procédé selon l'une des revendications 1 et 2, caractérisé en ce qu'au moins un radical R du corps de départ de formule II est un radical nitro et au moins un radical R du produit de formule I est un radical amino.

5. Procédé selon l'une quelconque des revendications 1 à 4, caractérisé en ce que l'hydrogénation est effectuée en présence de métaux du huitième sous-groupe de la classification périodique, déposé sur un support.

6. Procédé selon l'une quelconque des revendications 1 à 5, caractérisé en ce que l'hydrogénation est effectuée dans l'eau ou dans un solvant miscible à l'eau.

7. Procédé selon l'une quelconque des revendications 1 à 6, caractérisé en ce que l'hydrogénation est effectuée sous une pression pouvant aller jusqu'à 300 bar et à des températures de 0 à 200 °C.

8. Procédé selon l'une quelconque des revendications 1 à 7, caractérisé en ce que l'hydrogénation est effectuée sous des pressions pouvant aller jusqu'à 100 bar et à des températures de 20 à 120 °C.

9. Procédé selon l'une quelconque des revendications 1 à 8, caractérisé en ce que l'hydrogénation est effectuée sous des pressions de 20 à 60 bar et à des températures de 30 à 80 °C.

10. Procédé selon l'une quelconque des revendications 1 à 9, caractérisé en ce que l'hydrogénation est effectuée en continu.